(19) Europäisches Patentamt / European Patent Office / Office européen des brevets

(11) **EP 3 721 231 B1**

(12) **EUROPEAN PATENT SPECIFICATION**

(45) Date of publication and mention of the grant of the patent:
**12.01.2022 Bulletin 2022/02**

(21) Application number: **19730777.0**

(22) Date of filing: **14.06.2019**

(51) Int Cl.:
*G01N 33/569* (2006.01)     *G01N 33/487* (2006.01)
*G01N 33/49* (2006.01)     *G01N 33/68* (2006.01)

(86) International application number:
**PCT/EP2019/065724**

(87) International publication number:
**WO 2020/011487 (16.01.2020 Gazette 2020/03)**

(54) **METHOD OF OBTAINING AN INDICATOR OF PATIENT SEPSIS AND APPARATUS FOR SUCH A METHOD**

VERFAHREN ZUM ERHALTEN EINES INDIKATORS FÜR SEPSIS EINES PATIENTEN UND VORRICHTUNG FÜR SOLCH EIN VERFAHREN

MÉTHODE D'OBTENTION D'UN INDICATEUR DE SEPSIE CHEZ UN PATIENT ET APPAREIL CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.07.2018 GB 201811461**

(43) Date of publication of application:
**14.10.2020 Bulletin 2020/42**

(73) Proprietor: ZELLMECHANIK DRESDEN GmbH
01307 Dresden (DE)

(72) Inventors:
• **HEROLD, Christoph**
  **91052 Erlangen (DE)**
• **KLAUE, Daniel**
  **01277 Dresden (DE)**

(74) Representative: **Hoffmann Eitle**
**Patent- und Rechtsanwälte PartmbB**
**Arabellastraße 30**
**81925 München (DE)**

(56) References cited:
**WO-A1-2015/024690     WO-A2-2015/156876**

• **Joel Jämsä: "FLOW CYTOMETRIC ANALYSIS OF LEUKOCYTE SURFACE MOLECULE EXPRESSION IN CRITICAL ILLNESS COMPARISON BETWEEN SEPTIC AND NON-SEPTIC PATIENTS", , 1 January 2017 (2017-01-01), XP055605992, Retrieved from the Internet: URL:http://jultika.oulu.fi/files/isbn97895 26215778.pdf [retrieved on 2019-07-16]**
• **TOEPFNER ET AL.: "Detection of human disease conditions by single-cell morpho-rheological phenotyping of blood", ELIFE, vol. 7, E29213, 13 January 2018 (2018-01-13), pages 1-22, XP055605374, DOI: 10.7554/eLife.29213 cited in the application**
• **Valeri P. Maltsev ET AL: "Optics of White Blood Cells: Optical Models, Simulations, and Experiments : Methods and Disease Diagnoses" In: "Advanced Optical Flow Cytometry : Methods and Disease Diagnoses", 20 April 2011 (2011-04-20), Wiley-VCH Verlag GmbH & Co. KGaA, Weinheim, Germany, XP055605871, ISBN: 978-3-527-40934-1 pages 63-93, DOI: 10.1002/9783527634286.ch4, the whole document**

EP 3 721 231 B1

**Description**

Technical Field

The present invention relates to a method of obtaining an indicator of patient sepsis. It also relates to an apparatus configured for carrying out such a method.

Technical Background

Patient sepsis is a major concern in hospital environments. It is a life-threatening condition that arises when the body's response to infection causes injury to its own tissues and organs. This injury to the tissues and organs can lead to serious complications, including patient death. To understand the significance of this condition, it is worth noting that, in 2013, about as many people died from sepsis in Germany as died from heart attacks. It is therefore clear that monitoring for sepsis is highly important in hospital environments.

One current way of monitoring for sepsis is to measure the procalcitonin (PCT) level in blood plasma. This level is obtained by analyzing a blood sample from a patient. The higher the PCT-level, the more serious the sepsis. The PCT-level is a generally accepted sepsis marker, and it is also used to manage antibiotic therapy, as is reported in "Procalcitonin in sepsis and systemic inflammation: a harmful biomarker and a therapeutic target", British Journal of Pharmacology (2010) 159, 253-264.

Tests to monitor the PCT level are comparatively expensive and require taking additional blood samples. Therefore, in most cases, PCT testing is carried out only at strong suspicion of an infection. A possible combination of a standard blood test like the differential blood count of leukocytes and a test for monitoring the severity of an infection could reduce testing efforts and costs of such a procedure and thereby lead to extended use of infection tests in patients. This can ultimately result in earlier detection of infections. This is of the utmost importance in case of sepsis where the chance of survival decreases by 7.6 % for every hour delay in starting efficient treatment as reported in "Duration of hypotension before initiation of effective antimicrobial therapy is the critical determinant of survival in human septic shock", Kumar et al., Critical Care Medicine (2006) 34, 1589-1596.

J. Jämsä, "FLOW CYTOMETRIC ANALYSIS OF LEUKOCYTE SURFACE MOLECULE EXPRESSION IN CRITICAL ILLNESS - Comparison between septic and non-septic patients" is further prior art.

Summary of the Invention

The present invention aims at solving or at least alleviating the issues mentioned above. The invention is defined by the method according to claim 1 and by the apparatus according to claim 13. Preferred embodiments are defined in the dependent claims.

According to claim 1, a method of obtaining an indicator of patient sepsis involves imaging a blood sample using a device arranged for obtaining an image of individual blood cells within the sample. That is, the device obtains essentially a photographic image. In particular, this image is obtained in image space, rather than Fourier space. Put yet another way, what one obtains is in essence a photograph of the blood cells. That is, in the image which is obtained, one can distinguish between blood cells and other components of the blood, and one can obtain details as regards the shape and in particular the optical properties of the individual blood cells, including in particular the leukocytes. The image which is obtained needs to have a high enough resolution to allow for some imaging of the interior of the cell - i.e. the cells to be imaged need to be resolved beyond being mere pixels.

From that image, the optical properties of (preferably only) the leukocytes are obtained. Leukocytes (also known as white blood cells) are a constituent part of human or animal blood and respond in particular to an infection. The present inventors have discovered that such cells respond strongly to sepsis and in particular change their optical properties, as will be discussed further below. It is not necessary to use all of the pixels of the leukocytes to be imaged for that purpose, and it may well be sufficient to only use a subsection of those pixels. For example, it may be sufficient to only look at a ring just inside a cell's perimeter.

By optical properties, properties such as, but not limited to, a mean or median brightness value or a standard deviation of the brightness values of the pixels of the image of the leukocytes are meant.

It is possible to distinguish the leukocytes from other components of the blood by their shape and size.

Those optical properties which have been obtained from the image are compared with reference values. For example, it could be checked whether the mean brightness is higher or lower than a certain predetermined value. This would then lead to an indicator of patient sepsis, in line with what the present inventors have discovered, as will be shown later. It is to be noted that while an indicator of patient sepsis is obtained, it is entirely possible that other methods of diagnosing the patient's condition would, in addition, also have to be performed.

The claimed method is based on the analysis of leukocytes and can thus be implemented in parallel to standard differential blood tests which makes it easier to implement and more efficient than additional laboratory testing for the PCT-value. It is therefore possible to reach an extended use accompanied by earlier infection detection and evaluation of the presence and also the severity of a sepsis.

The fact that infections have an effect on cellular components such as the cytoskeleton, the cell nucleus, cytoplasmic granules and other components and can hence, in principle be detected using optical methods, can be confirmed from "Mechanotransduction in neutrophil activation and deactivation", Ekpenyong et al., Biochimica et Biophysica Acta (2015) 1853, 3105-3116, "The use of flow cytometry to measure neutrophil function", van Eeden et al., Journal of Immunological Methods (1999) 22, 23-43, and "Function of the cytoskeleton in human neutrophils and methods for evaluation", Torres and Coates, Journal of Immunological Methods (1999) 232, 89-109.

The indicator of the severity of the sepsis can then be used to monitor the efficacy of a treatment of the patient (e.g. an antibiotic treatment).

In the present context, the term "indicator of patient sepsis" encompasses the determination of the presence or absence of sepsis within a patient the blood sample has been taken from. I.e., one determines whether the patient is suffering from sepsis or not. It may also involve that, in addition to that determination, it is also determined how severe that sepsis is - i.e. whether it is a mild or a critical sepsis. This can be obtained in the same way a PCT-value of blood is analysed. Further, in the same way, one can also monitor how a sepsis is progressing, i.e. whether a patient is getting better or is getting worse.

It is preferred that the optical properties of only some types of the leukocytes are used to obtain an indicator of patient sepsis. The present inventors have found out that some subpopulations of the leukocytes are particularly sensitive to sepsis and are particularly good markers for sepsis. In particular, this applies to lymphocytes, neutrophil granulocytes, monocytes, eosinophil granulocytes, and basophil granulocytes, which are also reflected in some of the further dependent claims. Since one only looks at specific subpopulations of the leukocytes, the value which is obtained becomes even more meaningful. It is to be expected that a more detailed specification and restriction on leukocyte subtypes like T cells (and their subpopulations), B cells, mature and immature neutrophil granulocytes and others will lead to similar observations.

It is particularly preferred if optical properties of two or more types of leukocytes are compared with each other to obtain an indicator of patient sepsis.

It has been discovered to be particularly advantageous if neutrophil granulocytes and monocytes are compared with each other. This comparison could for example be by means of subtracting their respective median brightness values or the respective standard deviations of the pixel values of those two types of white blood cells from each other and comparing that difference to a threshold value. Such a method is particularly sensitive.

Likewise, in a similar vein, other combinations of leukocyte subpopulations like lymphocytes and neutrophil granulocytes, or monocytes and eosinophil granulocytes, and others can be compared with each other.

It is according to the invention that the optical properties to be obtained include a measure of the variation of the brightness values of the pixels of the image of neutrophil granulocytes, monocytes and/or eosinophil granulocytes. This measure of variation is preferably the standard deviation of those pixel values. Such a standard deviation is easy to calculate and allows for a good diagnosis.

The optical properties to be obtained can also include a comparison of the average brightness of the pixels of the image of neutrophil granulocytes and/or eosinophil granulocytes with an average brightness of the background. This average brightness could be, for example, the arithmetic mean or, preferably, the median. Again, such a calculation is easy to implement.

The optical properties to be obtained can also be investigated for their correlation with non-optical properties of the same cells such as, but not only, the size of the cell. One way to evaluate such correlations can be found in the value of the slope of a linear regression to the data in the space of optical property and non-optical property. Other ways could be various correlation coefficients of the data.

It is preferred that the correlation of the average brightness of the neutrophil granulocytes with the values of the area of the same neutrophil granulocytes is measured by the linear regression slope of the data. Again, such a measure has been shown to be particularly good to implement.

Finally, the invention also resides in the apparatus as defined in claim 13. This apparatus combines the advantages recited previously.

Brief Description of the Drawings

Figs. 1 to 12 show plots of optical properties of leukocytes and their dependence with a measured PCT-level.

Figs. 13 and 14 show the dependence of the average cell brightness on the cell area of neutrophil granulocyte populations for a patient with low (Fig. 13) and high (Fig. 14) PCT-level.

Fig. 15 shows the degree of correlation between blood cell properties and a measured PCT-level.

Detailed Description of the Drawings

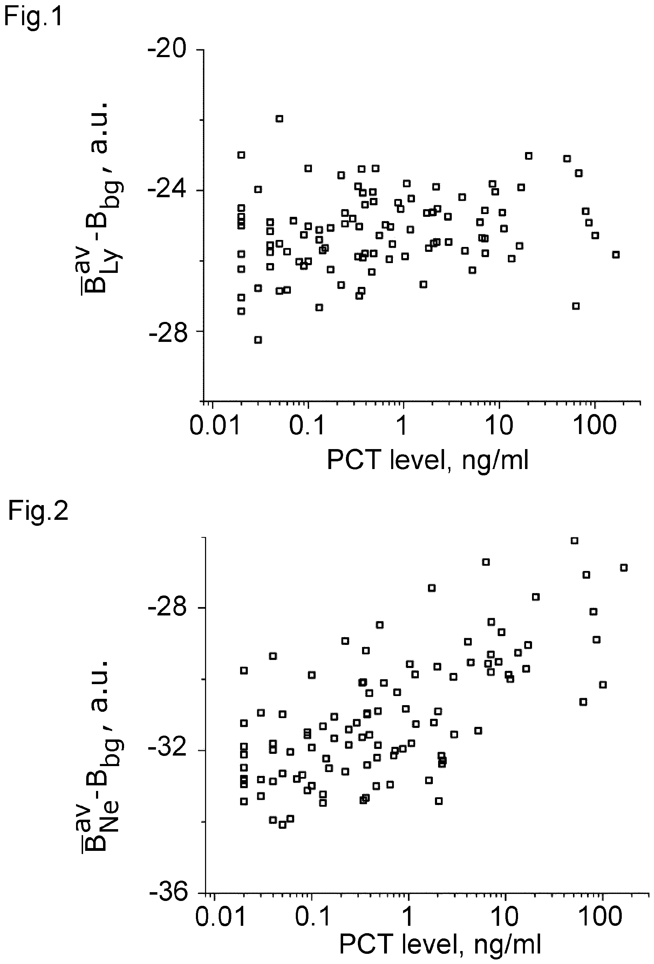

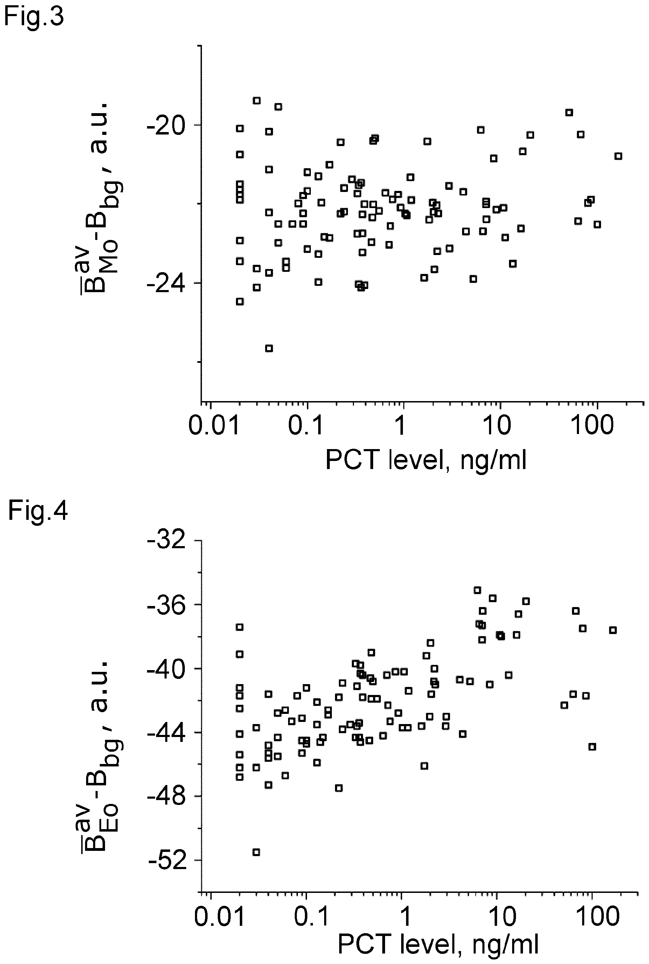

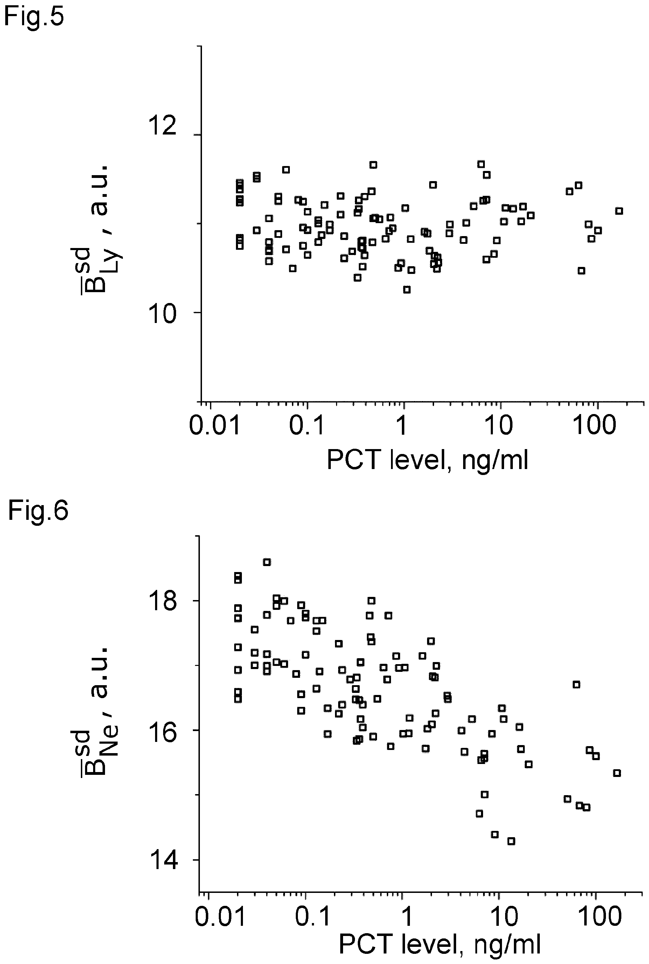

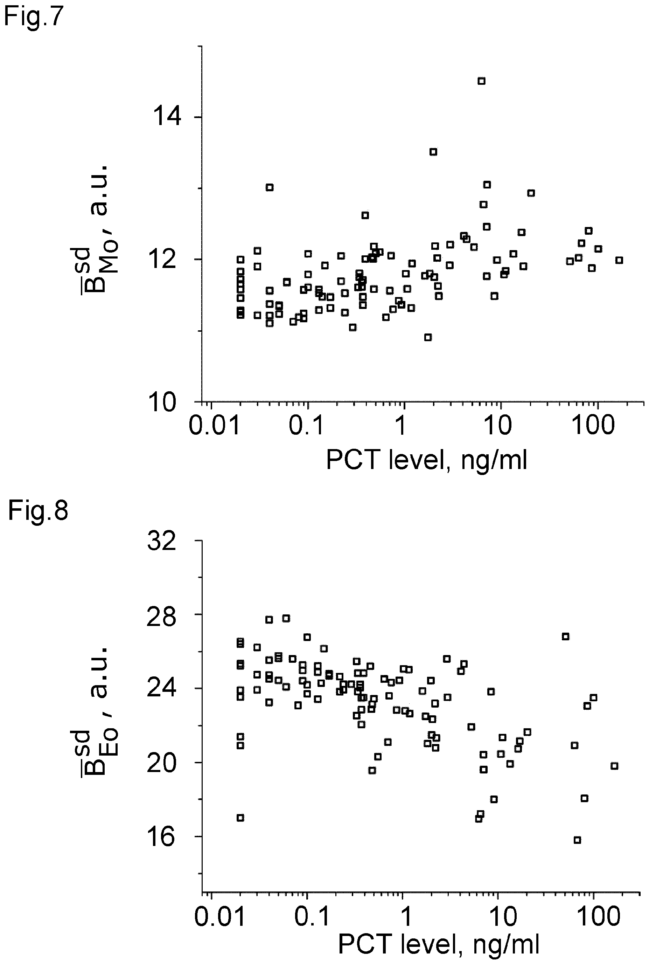

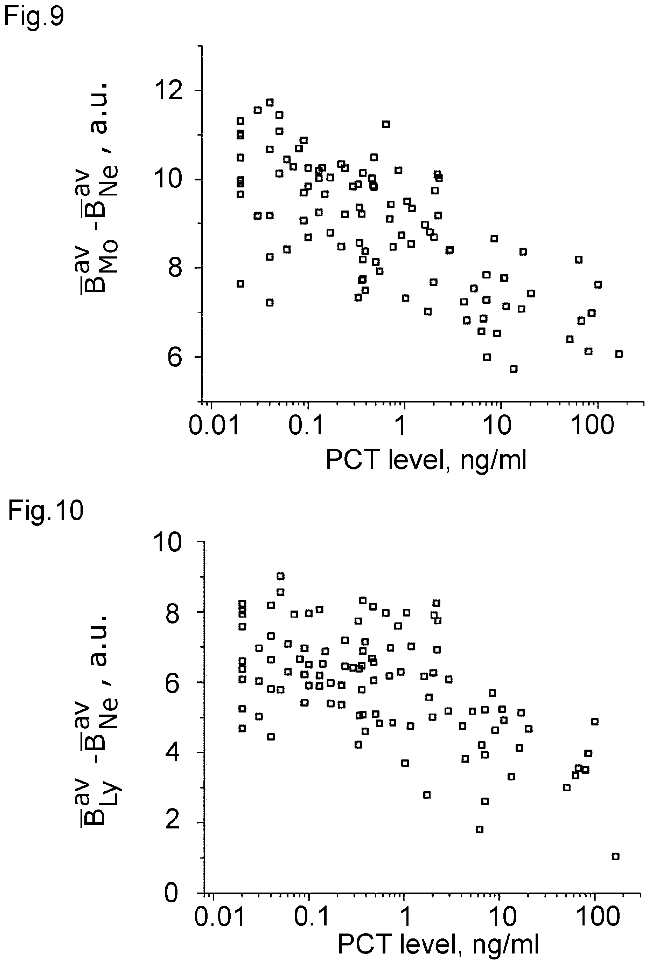

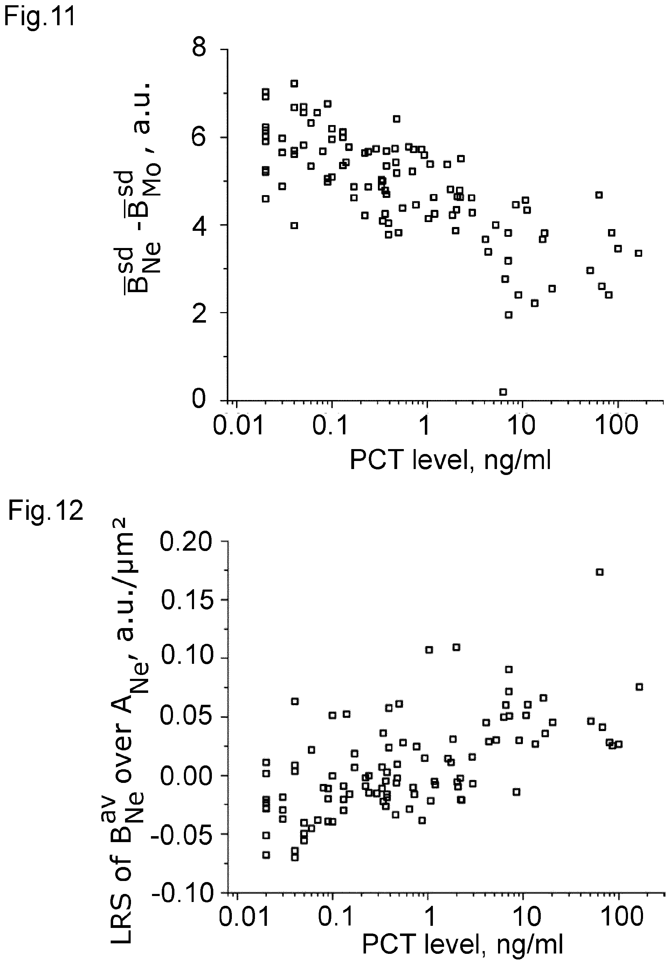

In the drawings of Figs. 1 to 15, experimental data are shown which show the results obtained when analyzing 103 leftover blood samples after standard clinical lab analytics were performed which involved the determination of PCT-levels. The leukocyte analysis method was carried out in line with what has been described for the leukocyte analysis in the first three subsections of the materials and methods section, in the description of Figure 1D in the main text and in Figure 1 - figure supplement 1 of "Detection of human disease conditions by single-cell morpho-rheological phenotyping of blood", Toepfner et al., eLife (2018) 7, e29213.

In that method, EDTA (ethylenediaminetetraacetic acid) anticoagulated whole blood samples which were diluted 1:50 in a cell carrier medium (based on 1x-PBS$^-$) and flushed through a 20 x 20 $\mu m^2$ channel at a total flow rate of 0.06 $\mu l/s$ (0.015 $\mu l/s$ sample flow, 0.045 $\mu l/s$ sheath flow) were imaged using a Zellmechanik Dresden GmbH AcCellerator device which is described in WO 2015/024690 A1.

The cells were imaged with a camera detecting standard bright field images using 2 $\mu s$ light flashes of a 460 nm LED. As an objective, an objective having a 40-times magnification and an NA of 0.65 was used. The inventors have performed further tests with a 640 nm LED or a white light LED and also a different objective.

Using the thus obtained data, the median brightness and the standard deviation of the brightness for the leukocytes were determined from all the pixel values determined to belong to the cell. It was possible to distinguish leukocytes from other components of the blood sample using the average brightness and the cell size. Using those parameters, it was also possible to distinguish between lymphocytes, monocytes, neutrophil granulocytes, eosinophil granulocytes, and basophil granulocytes. Further, measurements were obtained to ensure that the cells are correctly focused and in the focal plane.

Thus, the inventors found that any differences in the brightness values do not originate from cells travelling in different planes along the z-axis. It was thus determined that the cells are hydro-dynamically focused at the imaging plane (z-plane) in the channel with an accuracy of better than $\pm$ 250 nm. Depending on the positioning of the optical focus, the cell positions were focused to at least $\pm$ 2 $\mu m$ for the analysis method in the measurements. The typical optical focus of the objective in use was aligned about 2 to 3 $\mu m$ above the equatorial planes of the cells and the method works in a range of $\pm$ 6 $\mu m$ around the equatorial planes of the cells. Above numbers may change if a different optical setting is used that results in a changed depth of field, e.g., by using an objective of different numerical aperture.

After the cell type was determined, for every analyzed blood sample, the five leukocyte subpopulations and the erythrocytes (red blood cells) were separated in the analysis, so that one only obtains the statistical data for one type of cell. The inventors obtained for each of those subpopulations distribution data of a size measure (e.g. the area in the cell contour), a deformation measure (e.g. 1-circularity, or inertia ratio: $IR = I_{yy}/I_{xx}$, $I_{xx} = \iint y^2 dx\, dy$, $I_{yy} = \iint x^2 dx\, dy$), a measure related to an optical property dependent on the overall brightness value of the cell (e.g. the average brightness value for all pixels determined to belong to the cell), and a measure related to an optical property dependent on the spatial distribution of the brightness values of the cell (e.g. the standard deviation of the brightness values from all the pixels determined to belong to the cell).

Individual data distributions are characterized by the median value as a way of describing the center of the distribution. In particular, the following quantities are used:

$\overline{A}_{POP}$: median of the area in contour of all cells of the population POP

$IR_{POP}$: median of the inertia ratio of all cells of the population POP

$\overline{Deformation}_{POP}$: median of deformation (1-circularity) of all cells of the population POP

$\overline{B}_{POP}^{av}$ : median of the average brightness values of all cells of the population POP

$\overline{B}_{POP}^{sd}$ : median of the standard deviation brightness values of all cells of the population POP

To describe the variation of a distribution, the distribution width $\Delta^{68}$ giving the distance between 16th and 84th percentile (thus covering 68% of the data in the distribution around the distribution median) is used.

POP denotes the specific cell type:

Le... leukocytes (WBCs)

Ly... lymphocytes

Ne... neutrophil granulocytes

Mo... monocytes

Eo... eosinophil granulocytes

Ba... basophil granulocytes

Er... erythrocytes (RBCs)

The average background intensity of the image (arithmetic mean) is defined as a reference $B_{bg}$.

Within a cell population, several data distributions can form a multidimensional space. For example, the 2D space of $B_{Ne}^{av}$ and $A_{Ne}$ can be obtained. $B_{Ne}^{av}$ and $A_{Ne}$ are the values of average pixel brightness and area within the contour of every single neutrophil granulocyte of a sample. A measure to describe data behavior, or in other words, data correlation, in such a space can be found in the slope of a linear regression giving the linear regression slope LRS $B_{Ne}^{av}/A_{Ne}$.

To obtain some way of analyzing the blood samples of a patient, firstly, a correlation of the clinical laboratory's values of the PCT-level with the afore mentioned population metrics were obtained for the samples. Some such correlations as described by Kendall's tau including p-values are summarized in the tables reproduced below. The following table shows non-optical properties:

| population metric | correlation coefficient | p-value |
| --- | --- | --- |
| $\Delta^{68}\overline{A}_{Ne}$ | **0.45676** | 1.2E-11 |
| $\Delta^{68}\overline{A}_{Mo}$ | **0.54813** | 4.4E-16 |
| $\Delta^{68}\overline{Deformation}_{Er}$ | **0.45971** | 8.1E-12 |
| $\Delta^{68}\overline{IR}_{Er}$ | **0.49766** | 1.3E-13 |
| # of Le with A > 100 $\mu m^2$ | **0.5723** | 2.2E-16 |
| fraction of Le with A > 100 $\mu m^2$ | **0.52996** | 8.0E-15 |

Of note, the p-value is very low, thus showing that the correlations which are obtained have a very high statistical significance. Further, the correlations are also very strong.

It is considered that the amount of very large leukocytes is likely caused by higher fractions of immature cells such as myeloid precursor cells in the peripheral blood which are known to be present during infections.

Further, some optical data were measured and are summarized in the following table:

| population metric | correlation coefficient | p-value | Figure |
| --- | --- | --- | --- |
| $\overline{B}_{Ly}^{av} - B_{bg}$ | 0.17138 | 0.011 | 1 |
| $\overline{B}_{Ne}^{av} - B_{bg}$ | **0.44552** | 3.4E-11 | 2 |
| $\overline{B}_{Mo}^{av} - B_{bg}$ | 0.06556 | 0.33 | 3 |
| $\overline{B}_{Eo}^{av} - B_{bg}$ | **0.41852** | 5.7E-10 | 4 |
| $\overline{B}_{Ly}^{sd}$ | -0.05559 | 0.41 | 5 |

(continued)

| population metric | correlation coefficient | p-value | Figure |
|---|---|---|---|
| $\bar{B}^{sd}_{Ne}$ | -0.51798 | 1.3E-14 | 6 |
| $\bar{B}^{sd}_{Mo}$ | 0.37229 | 3.1E-8 | 7 |
| $\bar{B}^{sd}_{Eo}$ | -0.40473 | 1.8E-9 | 8 |
| $\bar{B}^{av}_{Mo} - \bar{B}^{av}_{Ne}$ | -0.49421 | 2.0E-13 | 9 |
| $\bar{B}^{av}_{Ly} - \bar{B}^{av}_{Ne}$ | -0.36692 | 4.8E-8 | 10 |
| $\bar{B}^{sd}_{Ne} - \bar{B}^{sd}_{Mo}$ | -0.55326 | 1.9E-16 | 11 |
| $LRS\ B^{av}_{Ne}/A_{Ne}$ | 0.44629 | 3.2E-11 | 12 |

The correlation coefficient denotes the correlation with the previously obtained PCT-level.

It can be clearly seen from the table that there is a strong correlation in the case of Figs. 2, 4, and 6 to 12, which can be used, with the corresponding threshold value, to obtain a measure of the PCT-level and, accordingly, sepsis. Again, it is clear that some of the correlations are strong enough to allow for inferring that those optical properties are a clear indicator of a PCT-level.

From these data, the inventors have learnt that white blood cells (leukocytes) and especially granulocytes (neutrophil and eosinophil) change their optical properties depending on the severity of a bacterial infection via the PCT-correlation. This applies to their optical properties linked to an average pixel brightness of an image of the cell (e.g. the arithmetic mean value) as well as the spacial variation of pixel brightness, as measured, e.g. by the standard deviation.

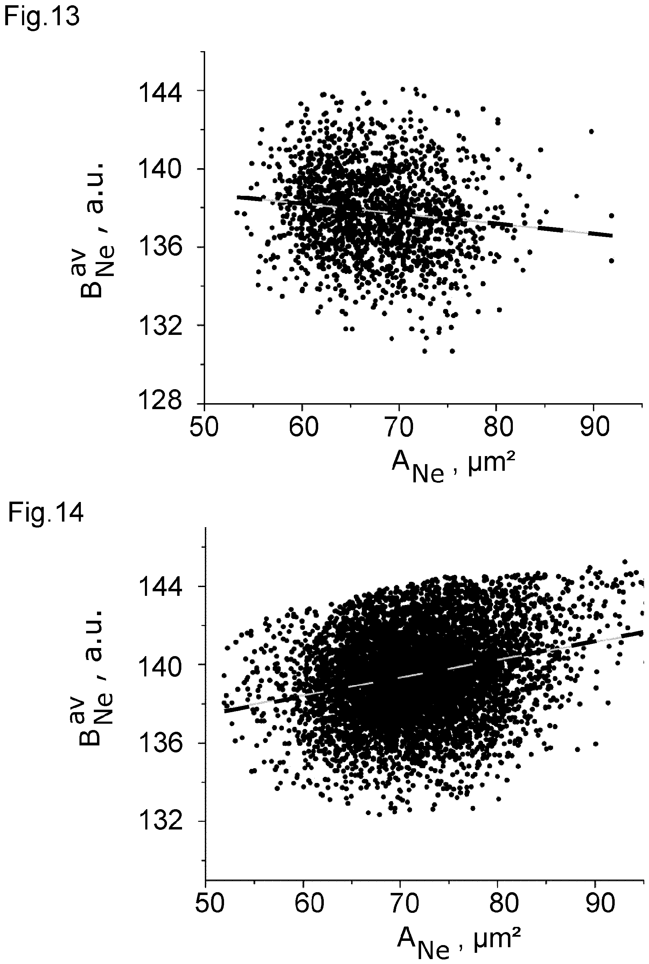

The best correlations to the PCT-value are obtained by comparing the properties of neutrophil granulocytes and monocytes, e.g. by calculating the difference in the average brightness and the brightness standard deviation (even though other values and measures are possible). The change in the neutrophil brightness is also revealed in the change of the dependence of the average brightness and the cell size from a negative correlation to a positive correlation, as can be seen from the second table. To illustrate this correlation between optical and non-optical properties, Fig. 13 shows an example of this dependence of the average brightness and the cell size of neutrophil granulocytes including the population data points and the linear regression curve of the data for a patient with a low PCT-level of 0.02 ng/ml and Fig. 14 shows and example for a patient with a high PCT-level of 7.08 ng/ml.

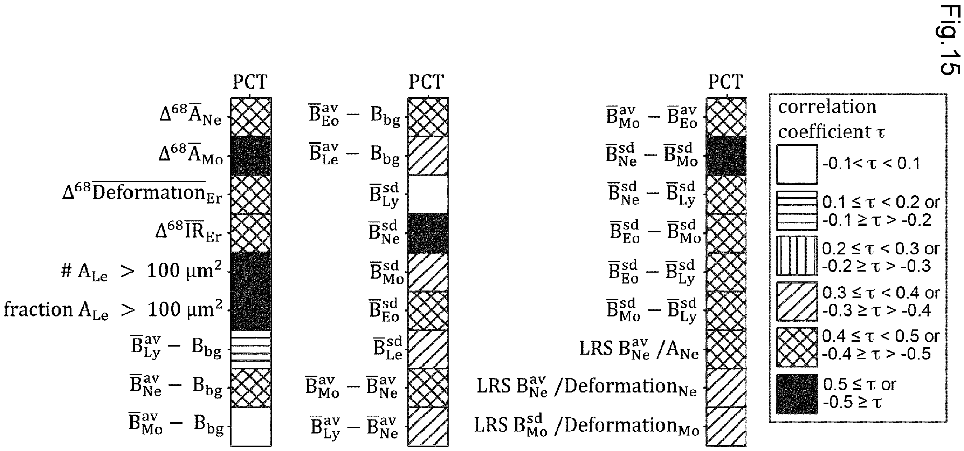

Fig. 15 shows a graphical representation of the strength of the correlation described by Kendall's tau of the aforementioned as well as further properties with the PCT-level.

Of note, it is not necessary to only look at one combination of values but it is also possible to analyse several combinations of values to determine the severity of an infection. These findings are also confirmed by what is shown in the "Automated determination of neutrophil VCS parameters in diagnosis and treatment efficacy of neonatal sepsis", Celik et al., Pediatric Research (2012) 71, 121-125. Similar results are also shown in "Volume Conductivity and Scatter Parameters as an Indicator of Acute Bacterial Infections by the Automated Haematology Analyser", Suresh et al., Journal of Clinical and Diagnostic Resarch (2016) 10, EC01-EC03; "Screening of sepsis using leukocyte cell population data from the Coulter automatic blood cell analyzer DxH800", Park et al., International Journal of Laboratory Hematology (2011) 33, 391-399, and "Quantitative Determination of Neutrophil VCS Parameters by the Coulter Automated Hematology Analyzer", Chaves et al., American Journal of Clinical Pathology (2005) 124, 440-444.

## Claims

1.  Method of obtaining an indicator of patient sepsis, comprising the following steps:

    - imaging a blood sample using a device arranged for obtaining an image of individual blood cells within the sample,
    - obtaining optical properties of leukocytes within the image,

- comparing the optical properties of the leukocytes with reference values so as to obtain an indicator of patient sepsis,

wherein optical properties to be obtained include a measure of the average brightness value of pixels of the image of the cells and/or wherein the optical properties to be obtained include a measure of the variation of the brightness values of pixels of the image of the cells.

2. Method according to claim 1, wherein optical properties of only a subsample of the leukocytes are used to obtain an indicator of patient sepsis.

3. Method according to claim 2, wherein a measure of the correlation of the optical properties with non-optical properties such as the cell size are used to obtain an indicator of patient sepsis.

4. Method according to claim 3, wherein the correlation of the optical properties and the non-optical properties include the calculation of the linear regression slope of the data.

5. Method according to claim 4, wherein the linear regression slope of the average brightness of the neutrophil granulocytes and the area of the neutrophil granulocytes is calculated.

6. Method according to one of claims 2 to 5, wherein optical properties of two or more types of leukocytes are compared with each other to obtain an indicator of patient sepsis.

7. Method according to claim 6, wherein neutrophil granulocytes and monocytes are compared with each other.

8. Method according to claim 6 or 7, wherein lymphocytes and neutrophil granulocytes are compared with each other.

9. Method according to one of claims 1 to 8, wherein the measure of the average brightness is the calculation of the arithmetic mean.

10. Method according to one of claims 1 to 9, wherein the cells are neutrophil granulocytes, monocytes and/or eosinophil granulocytes, wherein preferably, the measure of the variation of the brightness is the calculation of a standard deviation.

11. Method according to one of claims 2 to 10, wherein the optical properties to be obtained include a comparison of the average brightness of the pixels of the image of neutrophil granulocytes and/or eosinophil granulocytes with an average brightness of the background.

12. Method according to one of the preceding claims, wherein the imaging is performed so as to obtain an image of the leukocytes to be imaged in image space.

13. Apparatus configured to carry out the method according to one of the preceding claims.


**Patentansprüche**

1. Verfahren zum Erhalten eines Indikators von Sepsis eines Patienten, umfassend die folgenden Schritte:

- Abbilden einer Blutprobe unter Verwendung einer zum Erhalten eines Bilds von individuellen Blutzellen innerhalb der Probe angeordneten Vorrichtung,
- Erhalten von optischen Eigenschaften von Leukozyten innerhalb des Bilds,
- Vergleichen der optischen Eigenschaften der Leukozyten mit Referenzwerten, um einen Indikator von Sepsis eines Patienten zu erhalten,

wobei zu erhaltende optische Eigenschaften eine Messung des durchschnittlichen Helligkeitswerts von Pixeln des Bilds der Zellen einschließen und/oder wobei die zu erhaltenden optischen Eigenschaften eine Messung der Variation der Helligkeitswerte von Pixeln des Bilds der Zellen einschließen.

2. Verfahren nach Anspruch 1, wobei optische Eigenschaften von ausschließlich einer Teilprobe der Leukozyten verwendet werden, um einen Indikator von Sepsis eines Patienten zu erhalten.

3.  Verfahren nach Anspruch 2, wobei eine Messung der Korrelation der optischen Eigenschaften mit nicht-optischen Eigenschaften wie der Zellgröße verwendet werden, um einen Indikator von Sepsis eines Patienten zu erhalten.

4.  Verfahren nach Anspruch 3, wobei die Korrelation der optischen Eigenschaften mit den nicht-optischen Eigenschaften die Berechnung der Steigung der linearen Regression der Daten einschließen.

5.  Verfahren nach Anspruch 4, wobei die Steigung der linearen Regression der durchschnittlichen Helligkeit der neutrophilen Granulozyten und die Fläche der neutrophilen Granulozyten berechnet wird.

6.  Verfahren nach einem der Ansprüche 2 bis 5, wobei optische Eigenschaften von zwei oder mehr Arten von Leukozyten miteinander verglichen werden, um einen Indikator von Sepsis eines Patienten zu erhalten.

7.  Verfahren nach Anspruch 6, wobei neutrophile Granulozyten und Monozyten miteinander verglichen werden.

8.  Verfahren nach Anspruch 6 oder 7, wobei Lymphozyten und neutrophile Granulozyten miteinander verglichen werden.

9.  Verfahren nach einem der Ansprüche 1 bis 8, wobei die Messung der durchschnittlichen Helligkeit die Berechnung des arithmetischen Mittelwerts ist.

10. Verfahren nach einem der Ansprüche 1 bis 9, wobei die Zellen neutrophile Granulozyten, Monozyten und/oder eosinophile Granulozyten sind, wobei bevorzugt die Messung der Variation der Helligkeit die Berechnung einer Standardabweichung ist.

11. Verfahren nach einem der Ansprüche 2 bis 10, wobei die zu erhaltenden optischen Eigenschaften einen Vergleich der durchschnittlichen Helligkeit der Pixel des Bilds von neutrophilen Granulozyten und/oder eosinophilen Granulozyten mit einer durchschnittlichen Helligkeit des Hintergrunds einschließen.

12. Verfahren nach einem der vorstehenden Ansprüche, wobei das Abbilden durchgeführt wird, um ein Bild der im Bildraum abzubildenden Leukozyten zu erhalten.

13. Einrichtung, konfiguriert, um das Verfahren nach einem der vorstehenden Ansprüche auszuführen.

**Revendications**

1.  Procédé d'obtention d'un indicateur de sepsie chez un patient, comprenant les étapes suivantes :

    - l'imagerie d'un échantillon de sang à l'aide d'un dispositif agencé pour obtenir une image de cellules sanguines individuelles au sein de l'échantillon,
    - l'obtention de propriétés optiques de leucocytes au sein de l'image,
    - la comparaison des propriétés optiques des leucocytes à des valeurs de référence de façon à obtenir un indicateur de sepsie chez un patient,

    dans lequel les propriétés optiques à obtenir incluent une mesure de la valeur de luminosité moyenne des pixels de l'image des cellules et/ou dans lequel les propriétés optiques à obtenir incluent une mesure de la variation des valeurs de luminosité des pixels de l'image des cellules.

2.  Procédé selon la revendication 1, dans lequel les propriétés optiques d'un sous-échantillon uniquement des leucocytes sont utilisées pour obtenir un indicateur de sepsie chez un patient.

3.  Procédé selon la revendication 2, dans lequel une mesure de la corrélation des propriétés optiques à des propriétés non optiques telles que la taille des cellules est utilisée pour obtenir un indicateur de sepsie chez un patient.

4.  Procédé selon la revendication 3, dans lequel la corrélation des propriétés optiques et des propriétés non optiques comprend le calcul de la pente de régression linéaire des données.

**5.** Procédé selon la revendication 4, dans lequel la pente de régression linéaire de la luminosité moyenne des granulocytes neutrophiles et la zone des granulocytes neutrophiles est calculée.

**6.** Procédé selon l'une des revendications 2 à 5, dans lequel les propriétés optiques de deux types de leucocytes ou plus sont comparées les unes aux autres afin d'obtenir un indicateur de sepsie chez un patient.

**7.** Procédé selon la revendication 6, dans lequel les granulocytes neutrophiles et les monocytes sont comparés les uns aux autres.

**8.** Procédé selon la revendication 6 ou 7, dans lequel les lymphocytes et les granulocytes neutrophiles sont comparés les uns aux autres.

**9.** Procédé selon l'une des revendications 1 à 8, dans lequel la mesure de la luminosité moyenne est le calcul de la moyenne arithmétique.

**10.** Procédé selon l'une des revendications 1 à 9, dans lequel les cellules sont des granulocytes neutrophiles, des monocytes et/ou des granulocytes éosinophiles, dans lequel de préférence, la mesure de la variation de la luminosité est le calcul d'un écart type.

**11.** Procédé selon l'une des revendications 2 à 10, dans lequel les propriétés optiques à obtenir comprennent une comparaison de la luminosité moyenne des pixels de l'image des granulocytes neutrophiles et/ou des granulocytes éosinophiles à une luminosité moyenne du fond.

**12.** Procédé selon l'une des revendications précédentes, dans lequel l'imagerie est réalisée de façon à obtenir une image des leucocytes à imager dans l'espace image.

**13.** Appareil conçu pour réaliser le procédé selon l'une des revendications précédentes.

Fig.1

Fig.2

Fig.3

Fig.4

Fig.5

Fig.6

Fig.7

Fig.8

Fig.9

Fig.10

Fig.11

Fig.12

**Fig.13**

**Fig.14**

Fig. 15

EP 3 721 231 B1

PCT

$\Delta^{68}\bar{A}_{Ne}$
$\Delta^{68}\bar{A}_{Mo}$
$\Delta^{68}\overline{Deformation}_{Er}$
$\Delta^{68}\overline{IR}_{Er}$
$\# A_{Le} > 100\ \mu m^2$
fraction $A_{Le} > 100\ \mu m^2$
$\bar{B}^{av}_{Ly} - B_{bg}$
$\bar{B}^{av}_{Ne} - B_{bg}$
$\bar{B}^{av}_{Mo} - B_{bg}$

PCT

$\bar{B}^{av}_{Eo} - B_{bg}$
$\bar{B}^{av}_{Le} - B_{bg}$
$\bar{B}^{sd}_{Ly}$
$\bar{B}^{sd}_{Ne}$
$\bar{B}^{sd}_{Mo}$
$\bar{B}^{sd}_{Eo}$
$\bar{B}^{sd}_{Le}$
$\bar{B}^{av}_{Mo} - \bar{B}^{av}_{Ne}$
$\bar{B}^{av}_{Ly} - \bar{B}^{av}_{Ne}$

PCT

$\bar{B}^{av}_{Mo} - \bar{B}^{av}_{Eo}$
$\bar{B}^{sd}_{Ne} - \bar{B}^{sd}_{Mo}$
$\bar{B}^{sd}_{Ne} - \bar{B}^{sd}_{Ly}$
$\bar{B}^{sd}_{Eo} - \bar{B}^{sd}_{Mo}$
$\bar{B}^{sd}_{Eo} - \bar{B}^{sd}_{Ly}$
$\bar{B}^{sd}_{Mo} - \bar{B}^{sd}_{Ly}$
LRS $B^{av}_{Ne}/A_{Ne}$
LRS $B^{av}_{Ne}/Deformation_{Ne}$
LRS $B^{sd}_{Mo}/Deformation_{Mo}$

correlation coefficient $\tau$

$-0.1 < \tau < 0.1$

$0.1 \le \tau < 0.2$ or $-0.1 \ge \tau > -0.2$

$0.2 \le \tau < 0.3$ or $-0.2 \ge \tau > -0.3$

$0.3 \le \tau < 0.4$ or $-0.3 \ge \tau > -0.4$

$0.4 \le \tau < 0.5$ or $-0.4 \ge \tau > -0.5$

$0.5 \le \tau$ or $-0.5 \ge \tau$

## REFERENCES CITED IN THE DESCRIPTION

### Patent documents cited in the description

- WO 2015024690 A1

### Non-patent literature cited in the description

- Procalcitonin in sepsis and systemic inflammation: a harmful biomarker and a therapeutic target. *British Journal of Pharmacology,* 2010, vol. 159, 253-264
- **KUMAR et al.** Duration of hypotension before initiation of effective antimicrobial therapy is the critical determinant of survival in human septic shock. *Critical Care Medicine,* 2006, vol. 34, 1589-1596
- **J. JÄMSÄ.** *FLOW CYTOMETRIC ANALYSIS OF LEUKOCYTE SURFACE MOLECULE EXPRESSION IN CRITICAL ILLNESS - Comparison between septic and non-septic patients*
- **EKPENYONG et al.** Mechanotransduction in neutrophil activation and deactivation. *Biochimica et Biophysica Acta,* 2015, vol. 1853, 3105-3116
- **VAN EEDEN et al.** The use of flow cytometry to measure neutrophil function. *Journal of Immunological Methods,* 1999, vol. 22, 23-43
- **TORRES ; COATES.** Function of the cytoskeleton in human neutrophils and methods for evaluation. *Journal of Immunological Methods,* 1999, vol. 232, 89-109
- **TOEPFNER et al.** Detection of human disease conditions by single-cell morpho-rheological phenotyping of blood. *eLife,* 2018, vol. 7, e29213
- **CELIK et al.** Automated determination of neutrophil VCS parameters in diagnosis and treatment efficacy of neonatal sepsis. *Pediatric Research,* 2012, vol. 71, 121-125
- **SURESH et al.** Volume Conductivity and Scatter Parameters as an Indicator of Acute Bacterial Infections by the Automated Haematology Analyser. *Journal of Clinical and Diagnostic Resarch,* 2016, vol. 10, EC01-EC03
- **PARK et al.** Screening of sepsis using leukocyte cell population data from the Coulter automatic blood cell analyzer DxH800. *International Journal of Laboratory Hematology,* 2011, vol. 33, 391-399
- **CHAVES et al.** Quantitative Determination of Neutrophil VCS Parameters by the Coulter Automated Hematology Analyzer. *American Journal of Clinical Pathology,* 2005, vol. 124, 440-444